# EUROPÄISCHE PATENTSCHRIFT

(11) **EP 0 158 201 B1**
(45) Veröffentlichungstag und Bekanntmachung des Hinweises auf die Patenterteilung: **01.08.1990**
(21) Anmeldenummer: 85103460.3
(22) Anmeldetag: 23.03.1985
(51) Int. Cl.: C12N 15/00

(54) **Hybridplasmide mit einem Streptomyceten- und einem Escherichia coli-Replicon**
Hybrid plasmid containing a streptomyces and an Escherichia coli replicon
Plasmide hybride ayant un constituant reproduisant des streptomycetes et Escherichia coli

(30) Priorität: 31.03.1984 DE 3412093
(43) Veröffentlichungstag der Anmeldung: 16.10.1985
(73) Patentinhaber: HOECHST AKTIENGESELLSCHAFT, 65926 Frankfurt am Main (DE)
(72) Erfinder: Wohlleben, Wolfgang, Dr., D-4800 Bielefeld 1 (DE); Muth, Günter, D-4800 Bielefeld 1 (DE); Pühler, Alfred, Prof. Dr., D-4800 Bielefeld 15 (DE)

(56) Entgegenhaltungen:
- EP-A- 0 035 914
- EP-A- 0 066 701
- EP-A- 0 070 522
- EP-A- 0 077 649
- EP-A- 0 085 548
- JOURNAL OF BACTERIOLOGY, Band 146, Nr. 1, April 1981, Seiten 360-368; J.L. SCHOTTEL et al.: "Cloning and expression in Streptomyces lividans of antibiotic resistance genes derived from Escherichia coli"
- CURR. TOP. MICROBIOL. IMMUNOL., Band 96, 1982, Seiten 69-95; K.F. CHATER et al.: "Gene cloning in streptomyces"

## Beschreibung

Die Erfindung betrifft eine Gruppe von mindestens zwei in Streptomyceten kompatiblen Pendelvektoren mit einem E. coli-Replicon und unterschiedlichen sich von den Plasmiden pSG2, pSG5 (erhältlich aus Streptomyces ghanaensis DSM 2932) und pSVH1 ableitenden Streptomyceten-Replicons, wobei sich vorzugsweise das E. coli-Replicon von einem Plasmid der Reihe pBR322, pACYC177 oder pACYC184 ableitet. Die Erfindung betrifft weiterhin die Verwendung von mindestens zweien der Replicons aus der Gruppe der Streptomyceten-Plasmide pSG2, pSG5 (erhältlich aus Streptomyces ghanaensis DSM 2932) und pSVH1 zur Konstruktion von untereinander kompatiblen Pendelvektoren mit einem E. coli-Replikon.

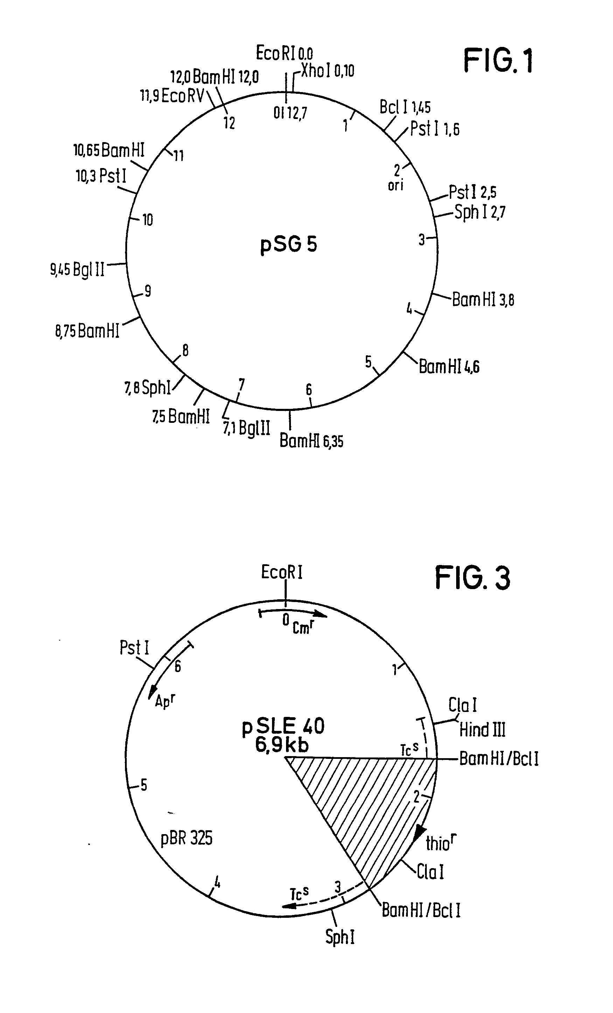

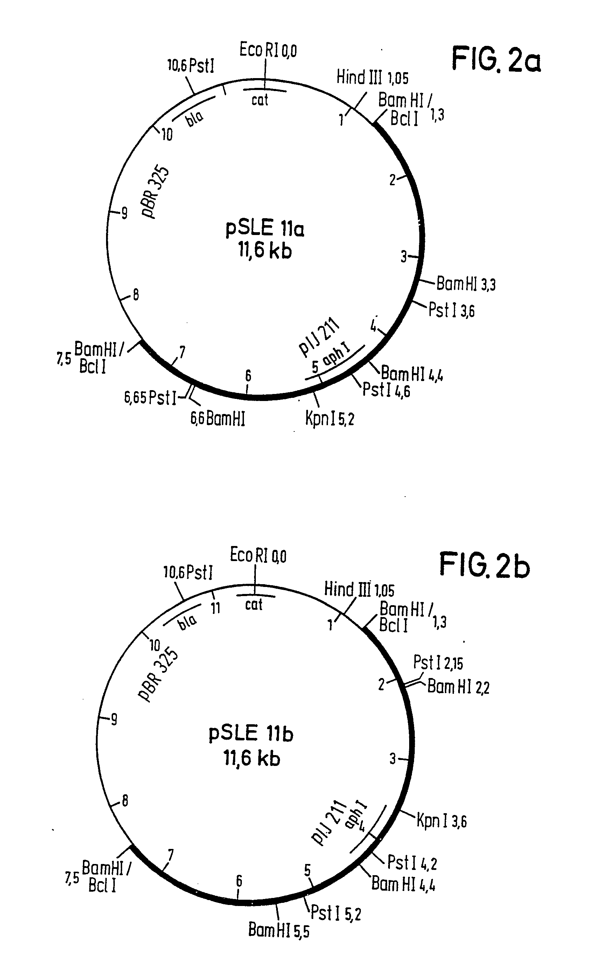

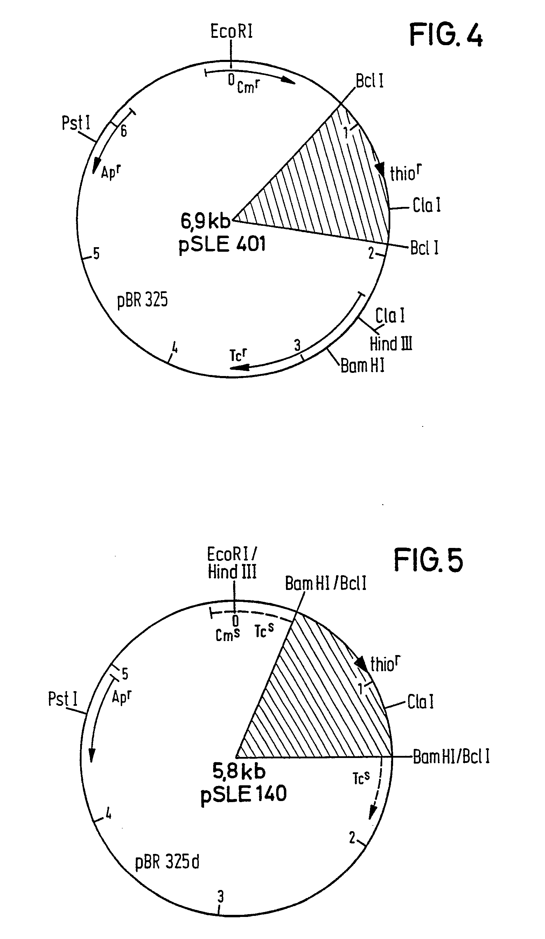

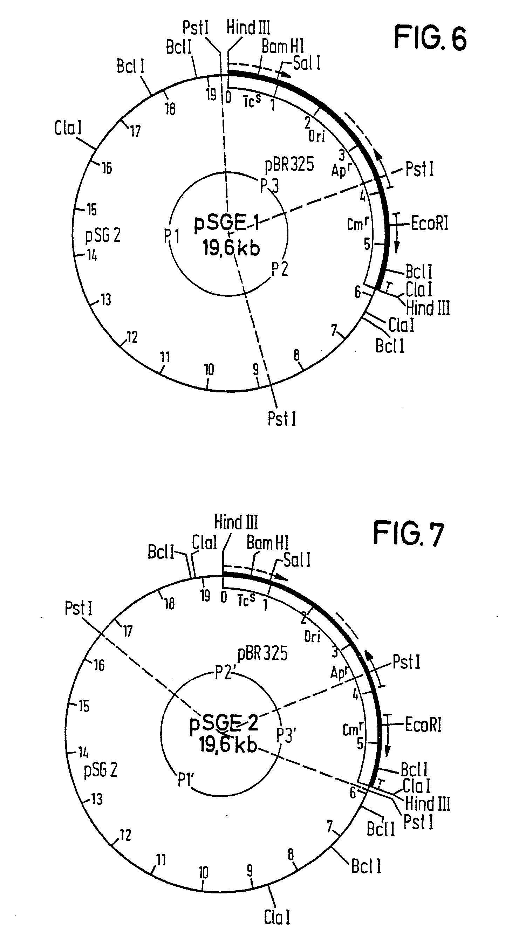

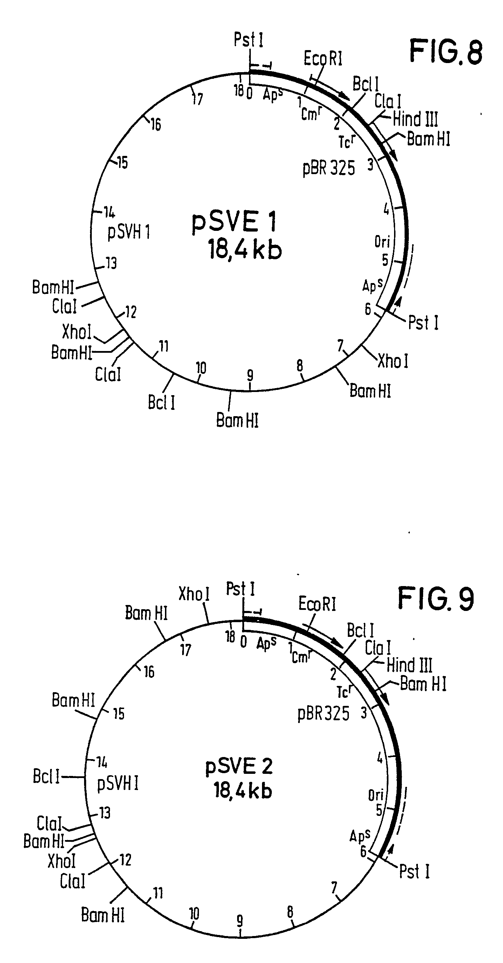

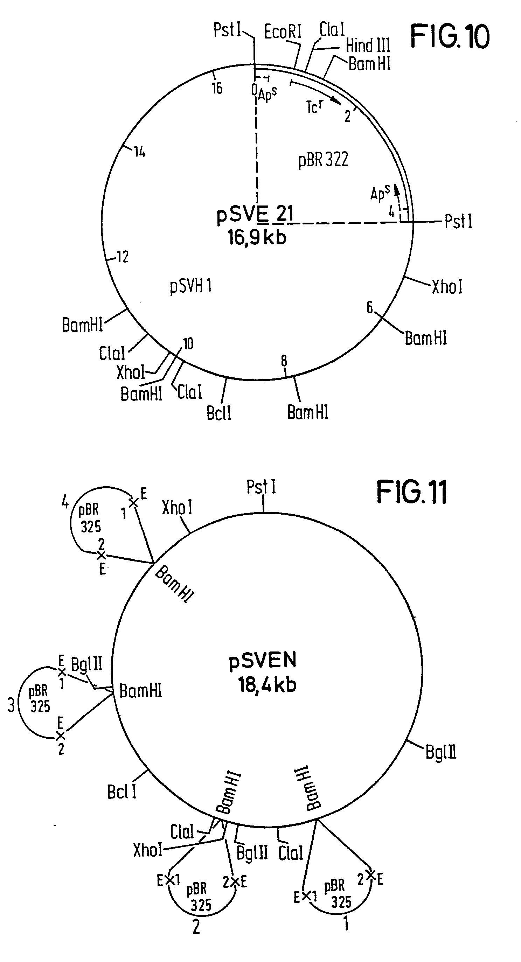

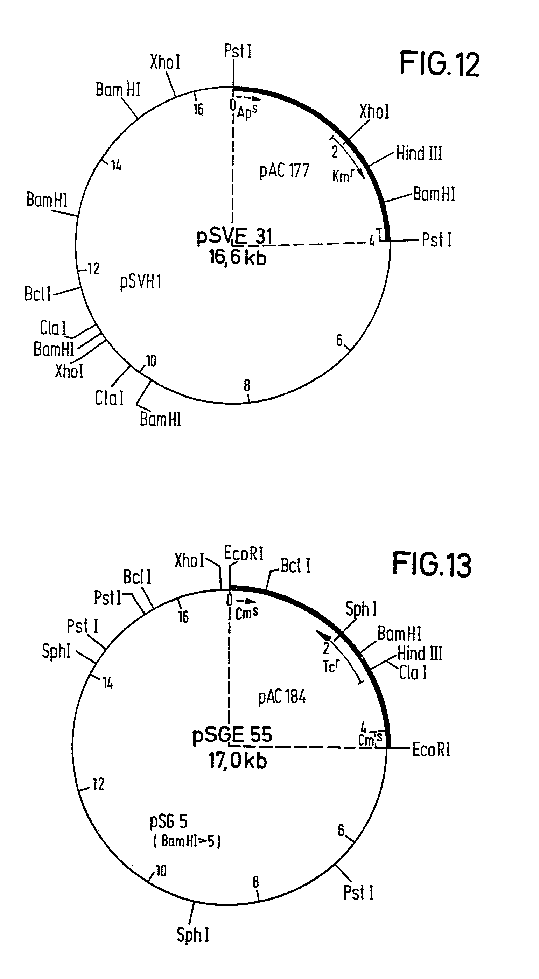

Das Streptomyceten-Plasmid pSG2 ist aus der EP-A 66 701 und das Streptomyceten-Plasmid pSVH1 aus der EP-A 70 522 bekannt. Das Streptomyceten-Plasmid pSG5 ist Gegenstand der am gleichen Tag eingereichten europäischen Anmeldung 85103461.1 (EP-A 158 872). Es ist durch die Figur 1 charakterisiert.

Die erfindungsgemäßen Pendelvektoren entstehen durch Fusion vom E. co/i-Plasmiden und den genannten Streptomyceten-Plasmiden und replizieren deshalb sowohl in E. coli als auch in Streptomyceten autonom. Weiterhin enthalten die erfindungsgemäßen Pendelvektoren Marker, vorzugsweise Resistenzgene, die eine Selektion in Streptomyceten und/oder in E. coli ermöglichen. Diese Pendelvektoren erlauben dadurch die Anwendung erprobter gentechnischer Methoden in E. coli, den Rücktransfer nach Streptomyceten und die Untersuchung und Nutzung manipulierter Gene in Streptomyceten. Weitere Aspekte und bevorzugte Ausgestaltungen der Erfindung werden im folgenden näher erläutert.

Als Ausgangsplasmide für den E. co/i-Anteil dienen bekannte E. co/i-Plasmide, vorzugsweise die aus der Literatur bekannten Plasmide pBR322 und pBR325 (Bolivar et al., Gene 2 (1977) 95; Bolivar, Gene 4 (1978) 121) und insbesondere die Plasmide der pAC-Reihe, pACYC177 und pACYC184 (Chang et al., J. Bacteriol. 134 (1978) 1141). Diese pAC-Plasmide eignen sich auf Grund ihrer geringen Größe von 4,0 bzw. 4,3 kb hervorragend zur Fusion mit Streptomyceten-Plasmiden.

In den erfindungsgemäßen Pendelvektoren können die E. co/i-DNA-Sequenzen unverändert vorliegen oder aber modifiziert sein, beispielsweise durch Einbau von Fremdgenen oder Regulationssequenzen. So kann durch Einbau weiterer E. co/i-Resistenzgene, beispielsweise durch Einbau eines Hindlll-Fragments aus dem Transposon Tn5 (Jorgensen et al., Mol. gen. Genet. 177 (1977) 65), das ein Gen für Neomycin/ Kanamycin-Resistenz trägt, in die Hindlll-Schnittstelle von pACYC184 ein geeignetes Ausgangsplasmid gewonnen werden, das die Bezeichnung pACYC184N (Tab. 3) erhielt. Selbstverständlich eignen sich für die erfindungsgemäßen Pendelvektoren auch in anderer Weise modifizierte E. co/i-Plasmide, in denen das ursprüngliche E. co/i-Replikationssystem erhalten bleibt. Als derart modifizierte E. coli-Plasmide gelten auch solche, in die ein weiteres E. co/i-Replikon inseriert ist, beispielsweise eines der vorstehend genannten Plasmide der pAC-Reihe.

Vorteilhaft für die Konstruktion der Pendelvektoren sind modifizierte E. co/i-Plasmide mit Resistenzgenen, die in Streptomyceten arbeiten. Diese Streptomyceten-Resistenzgene können auch in ein bereits modifiziertes E. co/i-Plasmid eingesetzt werden, beispielsweise kann in das Plasmid pBR325d (Tab. 1) die Thiostrepton-Resistenz aus dem Streptomyceten-Plasmid plJ6 (Thompson et al., Nature 286 (1980) 525) kloniert werden. Man erhält so ein modifiziertes E. co/i-Plasmid, pSLE140 genannt, das zur Konstruktion der erfindungsgemäßen Pendelvektoren verwendet werden kann.

Als weitere Beispiele zur Modifizierung der E. co/i-Plasmide mit Streptomyceten-Resistenzgenen seien genannt: Das Plasmid pSLE11, in das das Neomycin-Resistenzgen aus dem Streptomyceten-Plasmid plJ211 (Kieser et al., Mol. gen. Genet. 185 (1982) 223) kloniert wurde, sowie die Plasmide pSLE40 und 401, die das Thiostrepton-Resistenzgen aus dem Streptomyceten-Plasmid plJ6 tragen.

In der folgenden Tabelle 1 sind erfindungsgemäß verwendbare Vektoren aufgeführt, die durch Einbau von Resistenzgenen aus Streptomyceten-Plasmiden erhalten werden und zur Konstruktion von Pendelvektoren dienen können:

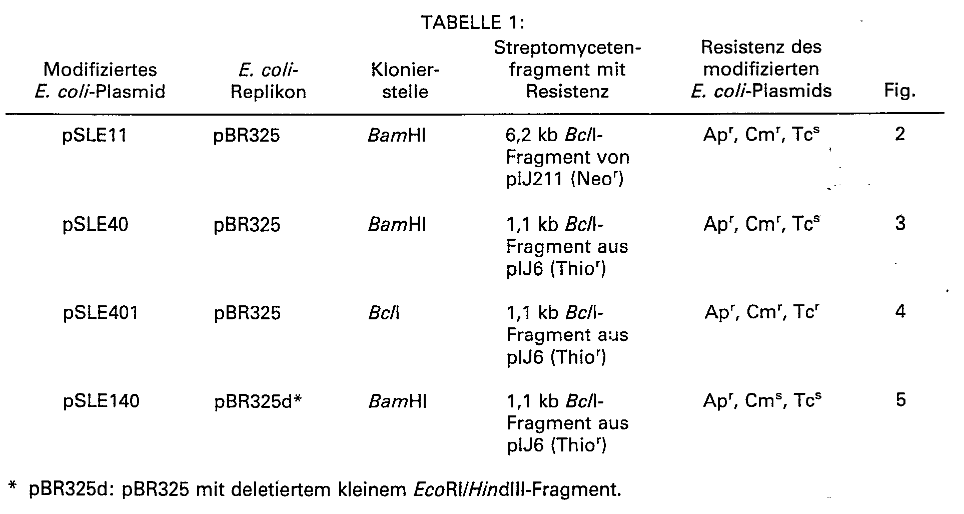

In der folgenden Tabelle 2 sind erfindungsgemäße Fusionsplasmide genannt:

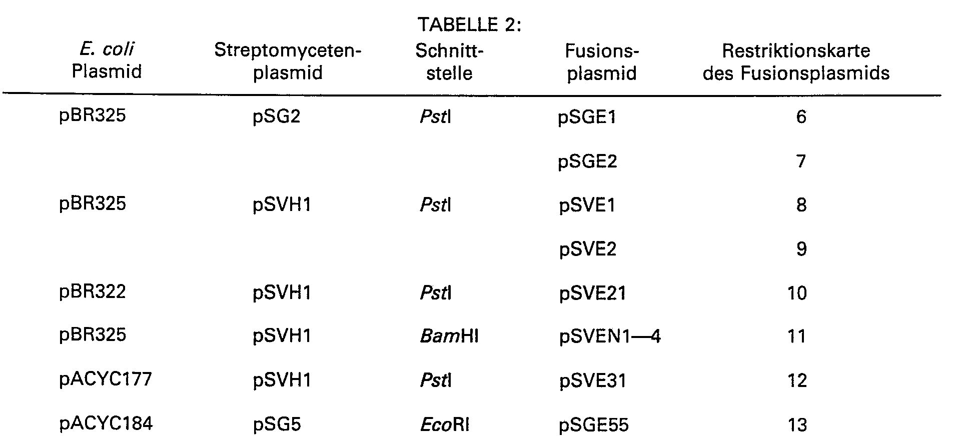

Diese Plasmide dienen einerseits zur vereinfachten Streptomyceten-DNA-Gewinnung aus E. coli, da sie in E. coli stabil sind und in der hohen Kopienzahl des E. coli-Plasmids vorliegen. Andererseits sind sie als Vorstufen zur Konstruktion von Pendelvektoren geeignet.

Die Tabelle 3 nennt erfindungsgemäße Pendelvektoren, die aus modifizierten E. co/i-Plasmiden (Tabelle 1) erhalten wurden und die auf dem E. coli-Replicon pBR325 basieren:

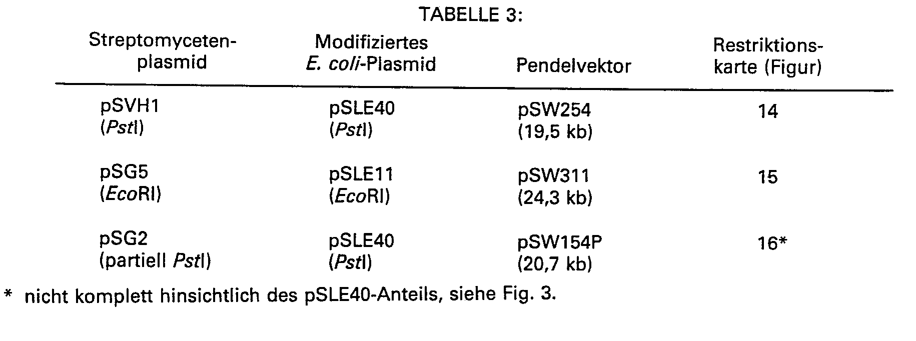

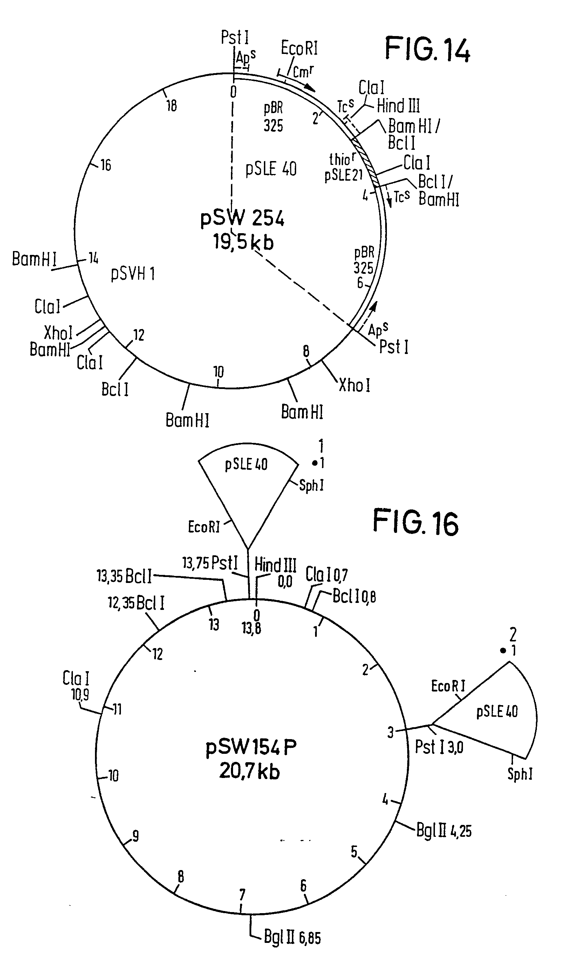

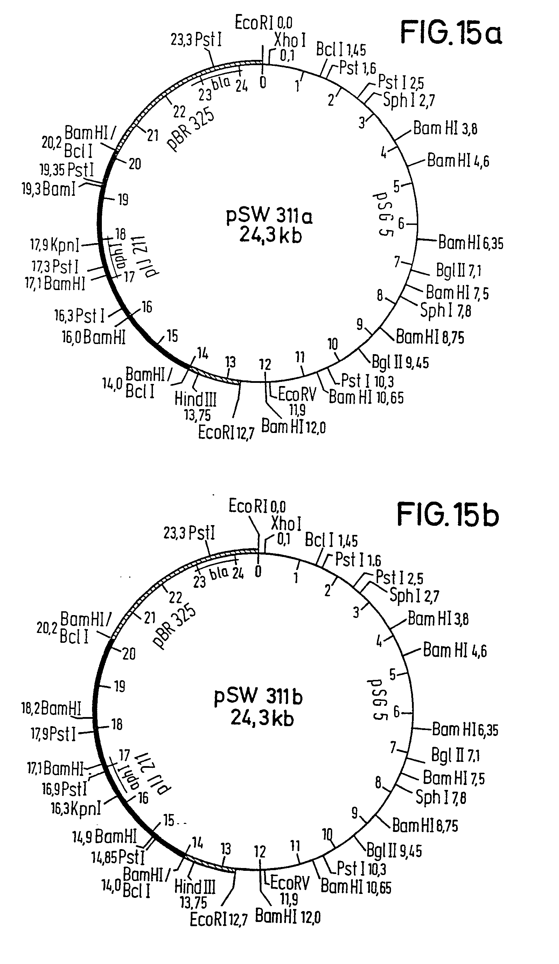

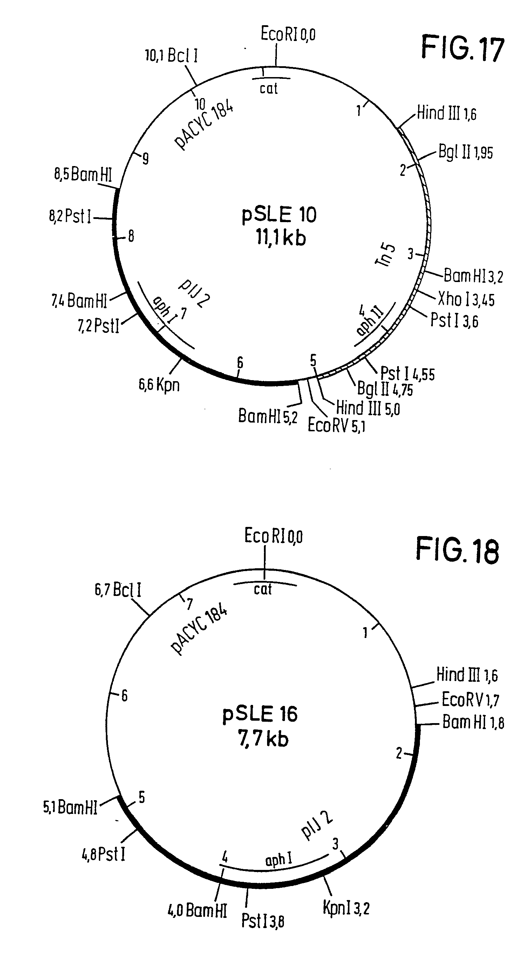

Für jeden Pendelvektor sind verschiedene Fusionierungsmöglichkeiten der Ausgangsplasmide gegeben. In Figur 16 sind 4 Varianten dargestellt. Bevorzugt angewandt wurde die Variante pSW154P2-2.

Die erfindungsgemäßen Pendelvektoren mit E. co/i-Replica der pBR-Reihe sind in E. coli in jedem Fall stabil; Deletionen im Streptomyceten-Plasmidanteil sind nicht beobachtet worden. In Streptomyces exprimieren pSW254, pSW311 und pSW154P die jeweilige Resistenz (Thiostrepton- bzw. Neomycinresistenz). Die Plasmide können nach S. lividans transformiert werden: ein Beweis dafür, daß die Replikationsgene nicht spezifisch für den ursprünglichen Wirt sind. Aufgrund der Kopienzahl (etwa 10) und der Kompatibilität des Replikons untereinander und mit pIJ101 und SLP1-2 (US-Patentschrift 4360597) sind die Pendelvektoren geeignet, bestehende Vektorsysteme so zu erweitern, daß komplexe Biosynthesewege schrittweise in Streptomyces kloniert werden können.

In Tabelle 4 sind modifizierte Plasmide der pAC-Reihe aufgeführt, die Resistenzgene zur Selektion in Streptomyceten tragen und die als Vorstufen zur Konstruktion von Pendelvektoren verwendet werden können:

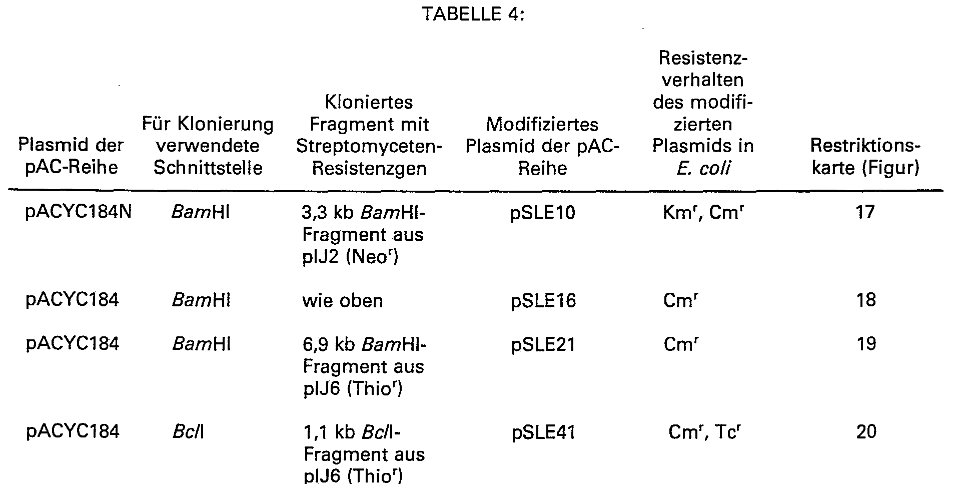

Unter Verwendung der in Tabelle 4 genannten modifizierten Plasmide der pAC-Reihe können u.a. die in der Tabelle 5 aufgeführten Pendelvektoren konstruiert werden:

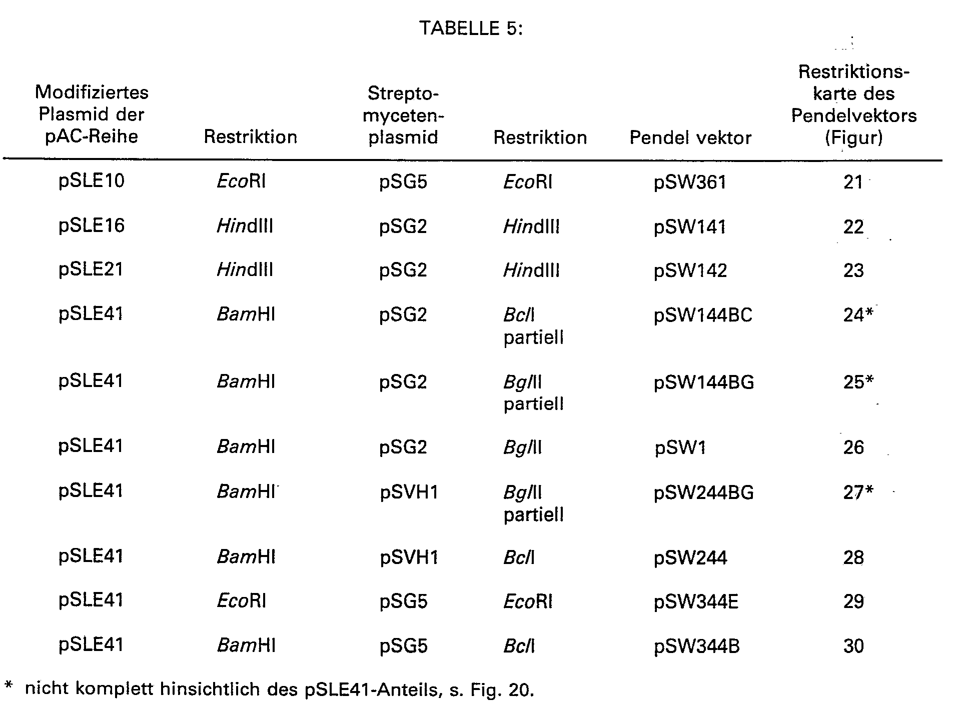

Für jeden Pendelvektor sind verschiedene Fusionierungsmöglichkeiten der Ausgangsplasmide gegeben. In den Figuren sind die Varianten dargestellt.

Besonders bevorzugte Pendelvektoren sind die Plasmide der Figuren 25 und 27, und zwar jeweils insbesondere diejenige Variante, bei der die Insertion pSLE41 mit der Ziffer 1 bezeichnet ist (Figur 27a), sowie die Plasmide der Figuren 26, 28 und 29.

Es ist selbstverständich auch möglich, den Streptomycetenplasmid-Anteil der erfindungsgemäßen Vektoren zu modifizieren, indem beispielsweise nicht-essentielle Regionen eliminiert werden. So wurde gefunden, daß beim Plasmid pSG5 die Replikationsregion auf dem größten BamHl-Fragment (4,45 kb) liegt, und zwar in dem Bereich von 1 bis 3 kb entsprechend Figur 1. Zur Konstruktion von Vektoren, die in Streptomyceten replizieren, genügt somit diese "essentielle Region".

Zur Konstruktion eines "Minimalreplicons" bietet sich z.B. das genannte 4,45 kb lange BamHl-Fragment an. Zirkularisiert man dieses Fragment, so erhält man ein Plasmid, das durch singuläre Schnittstellen für Bcll, Sphl, BamHl, EcoRl und Xhol ausgezeichnet ist. Für die vier letztgenannten Schnittstellen ist gezeigt, daß sie für Klonierungen zur Verfügung stehen. Darüber hinaus liegen auf diesem Fragment noch Schnittstellen für Pvull (bei 12,6, 3,2 und 3,6 kb gemäß Figur 1), die außerhalb der "essentiellen Region" liegen und somit für Klonierungen oder weitere Verkürzung(en) zur Verfügung stehen. Es wurden weiterhin Schnittstellen für Sstll (bei 0,6, 1,8 und 3,65 kb gemäß Figur 1) gefunden, von denen die mittlere vermutlich in der "essentiellen Region" liegt. Außerdem sind mindestens fünft Sa/I-Schnittstellen vorhanden.

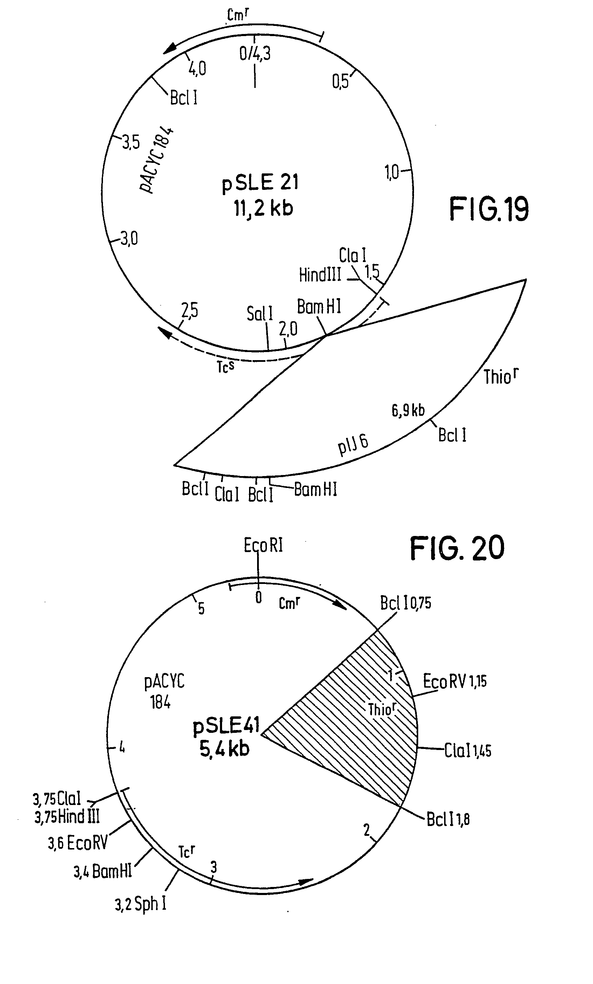

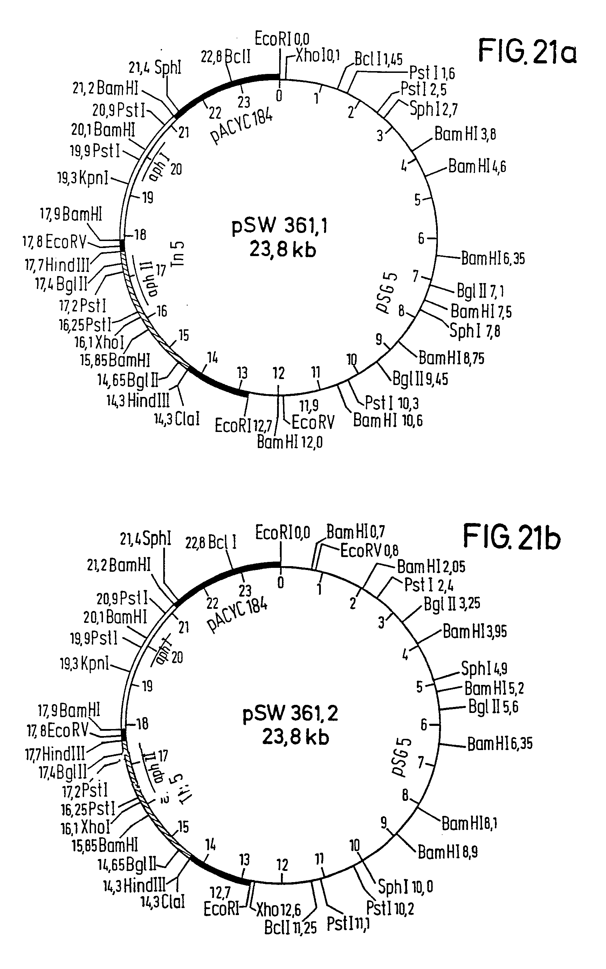

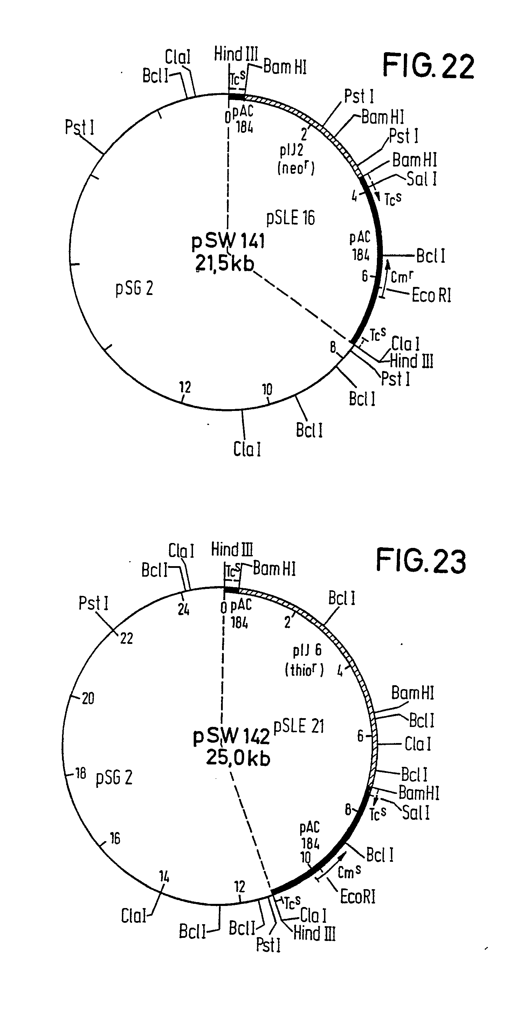

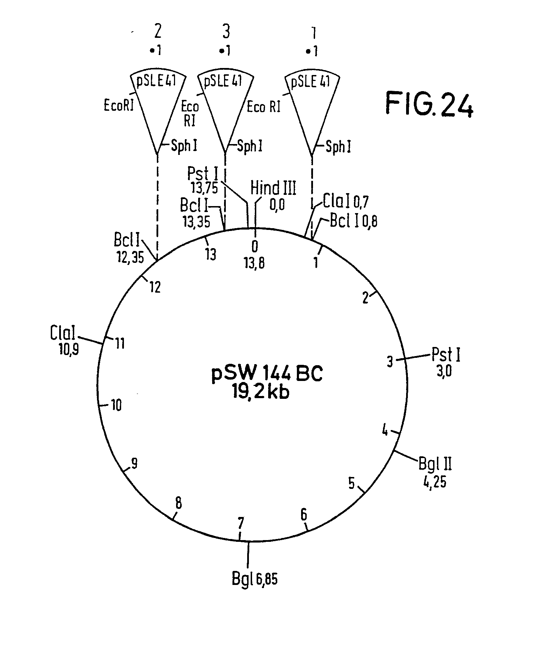

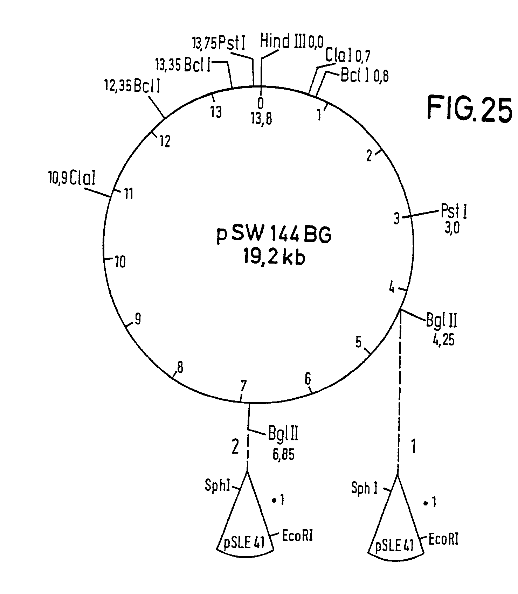

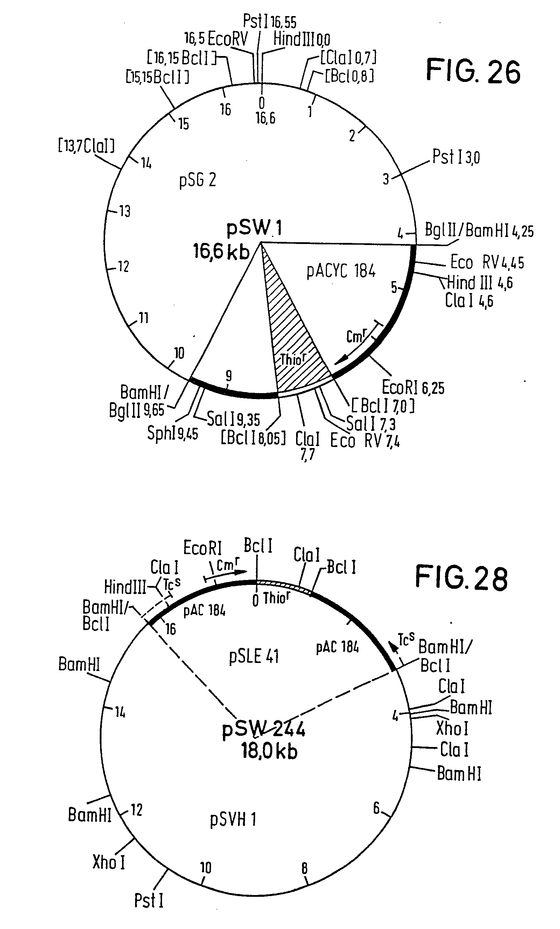

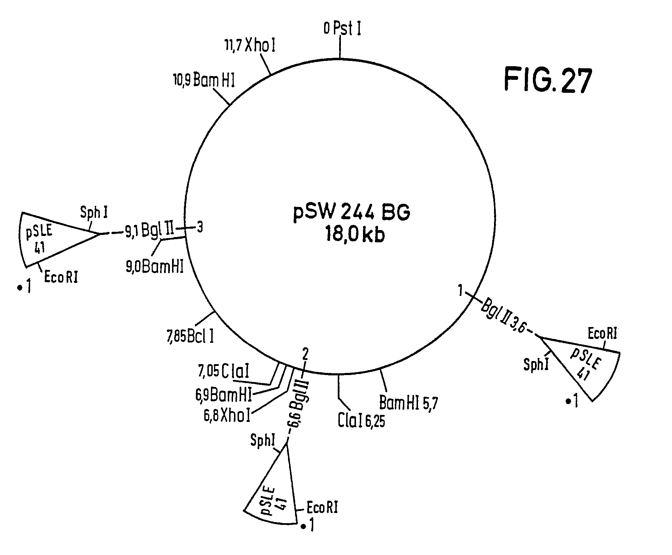

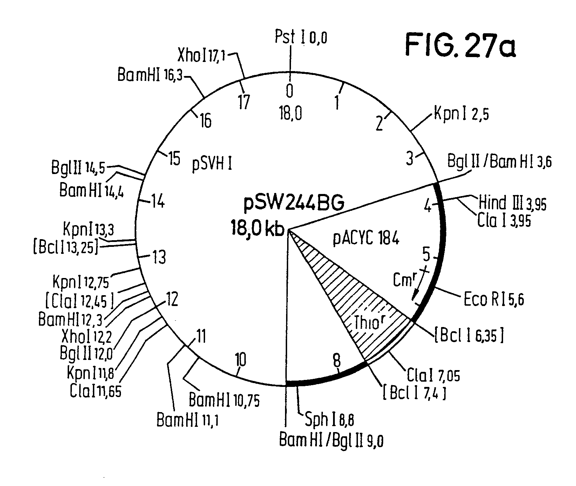

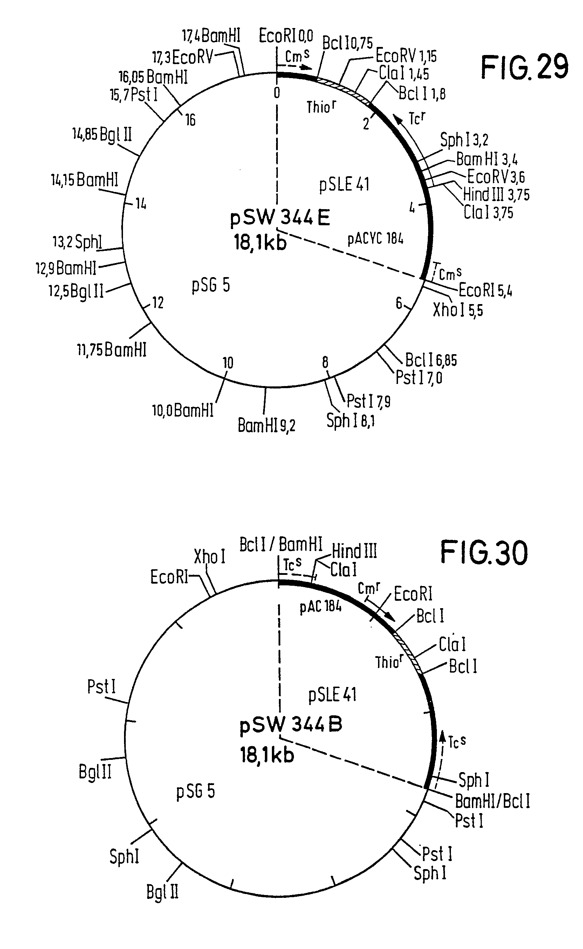

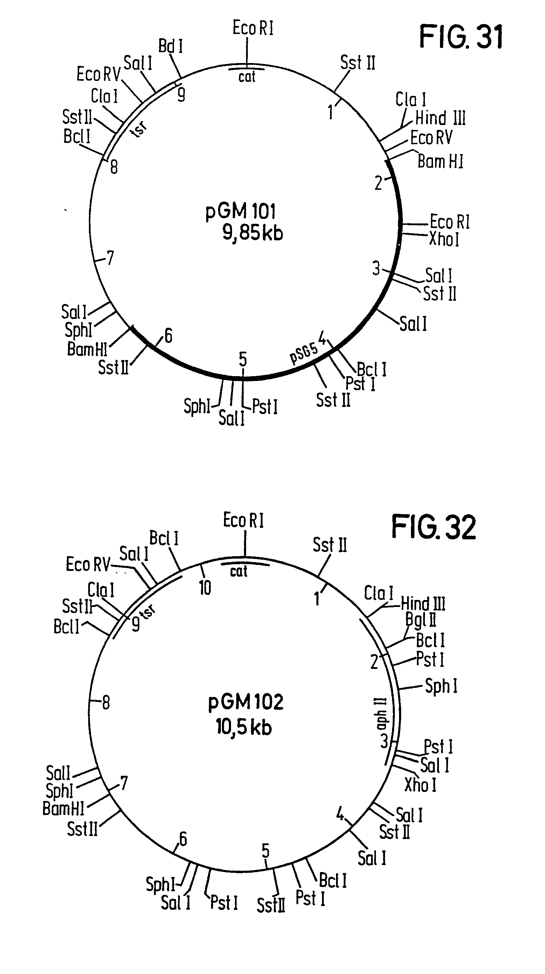

Wird beispielsweise das Plasmid pSLE41 (Figur 20) mit BamHl geöffnet und mit dem 4,45 kbBamHl-Fragment von pSG5 ligiert, so resultiert das Hybridplasmid pGM101 (Figur 31, in der im Replicon nur drei Sa/l-Schnittstellen kartiert sind). Dieses Plasmid verfügt u.a. über singuläre Schnittstellen für Hindlll und Xhol in nichtessentiellen Regionen. Zur Selektion stehen die Marker Chloramphenicol-Resistenz (in E. coli) und Thiostrepton-Resistenz (in Streptomyces) zur Verfügung. Die Molekülgröße beträgt 9,9 kb.

Eliminiert man aus pGM101 das 1 kb Hindlll-Xhol-Fragment und inseriert statt dessen das 1,6 kb Hindlll-Xhol-Fragment von Tn5, das das aphll-Resistenzgen für Neomycin trägt, so entsteht das Hybridplasmid pGM102 (Figur 32, in der im Replicon nur drei Sa/l-Schnittstellen kartiert sind). Dieses repliziert in E. coli und S. lividans. Zur Selektion in E. coli stehen die Chloramphenicol- und die Neomycinresistenz, in S. lividans die Thiostrepton- und die Neomycinresistenz zur Verfügung. Zur Klonierung können in E. coli die singulären Schnittstellen für EcoRl (Inaktivierung der Chloramphenicol- Resistenz) und Bg/II (Inaktivierung der Neomycin-Resistenz), in Streptomyceten die für EcoRV (Inaktivierung der Thiostrepton-Resistenz) und ebenfalls Bg/II dienen, außerdem die singulären Schnittstellen für Hindlll un Xhol.

Die erfindungsgemäßen Pendelvektoren mit einem pAC-Replicon sind in E. coli in jedem Fall stabil. Deletionen im Streptomyceten-Plasmidteil sind in E. coli nicht beobachtet worden. Die in Streptomycesarten ausführlich getesteten Vektoren (insbesondere pSW344E, pSW1 und pSW244BG) sind in den untersuchten Stämmen (u.a. S. lividans, S. geysirensis, S. ghanaensis und S. venezuelae) stabil und exprimieren die Streptomyceten-Resistenzgene.

Die entscheidenden Vorteile dieser Vektoren sind:
1. Der Wirtsbereich umfaßt auch Stämme (z.B. S. ghanaensis), die von bisher bekannten Vektoren nicht transformiert werden können.
2. Die Kopienzahl liegt mit etwa 10 in einer Größenordnung, die die bisher bekannten Vektoren nicht abdecken. Die Kopienzahl anderer Streptomyceten-Plasmide und deren Derivate werden mit 1 (SCP2^{*}, europäische Patentanmeldung mit der Veröffentlichungsnummer 92 388), 3―5 (SLP1.2) und mit 20 Kopien/ Zelle (z.B. für plJ101 u.a., Kieser, a.a.0.) angegeben.
3. Die Plasmide pSVH1, pSG2 und pSG5 sowie deren Derivate gehören zu unterschiedlichen Kompatibilitätsklassen. Zudem sind sie mit pIJ101- und SLP1.2-Replikons kompatibel. Somit ist es möglich, parallel unterschiedliche Gene auf verschiedenen Replikons in einer Zelle zu untersuchen, und zwar jeweils in ausgesuchter Kopienzahl.

Auf Grund ihrer vorteilhaften Eigenschaften eignen sich die erfindungsgemäßen Pendelvektoren nicht nur zur Untersuchung von Genen und zur Kartierung von Replikons (beispielsweise durch Transposon-Mutagenese in E. coli), sondern auch zur Veränderung und zum Transfer von Genen. Die Arbeitsweise ist allgemein bekannt und beispielsweise in den vorstehend genannten Patentdokumenten, den US-Patentschriften 4 332 900, 4 338 400, 4 340 674, 4 343 906, 4 362 816, 4 362 817, 4 393 137 und 4 401 761 sowie in dem Lehrbuch von Maniatis et al., Molecular Cloning, Cold Spring Harbor 1982, beschrieben. Die Erfindung betrifft somit auch die Verwendung der erfindungsgemäßen Pendelvektoren zur Expression von Fremdgenen in Streptomyceten.

In den folgenden Beispielen wird die Erfindung näher erläutert.

Zunächst wird die Isolierung des Plasmids pSG5 beschrieben:
Zur Kultivierung des Stammes S. ghanaensis DSM 2932 für eine anschließende Lyse sind folgende Medien geeignet:

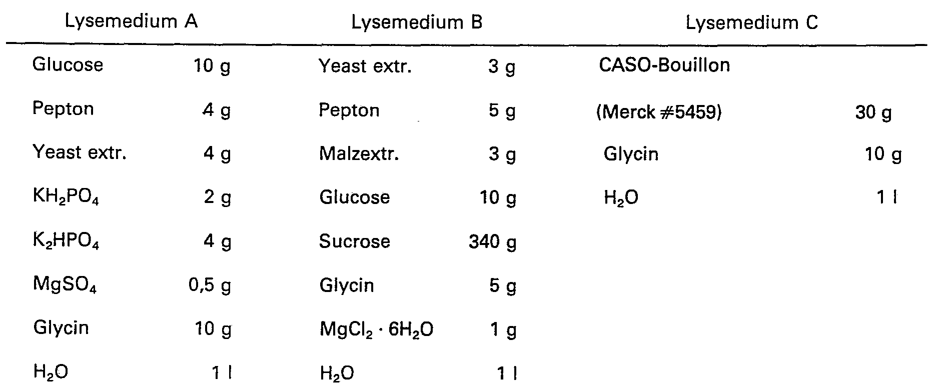

In einem 500 ml Erlenmeyerkolben werden ca. 100 ml Nährmedium mit einer homogenisierten Einzelkolonie beimpft und 2-3 Tage bei 30°C im Rundschüttler (120 rpm) inkubiert.

Ca. 50 ml einer 3 Tage alten, homogenisierten Flüssigkultur werden in JA-20 Bechern einer Beckman-Kühlzentrifuge J21 C geerntet (10 min, 10000 rpm, 25°C) und einmal in TESu (10 mM Tris. HCI, 1 mM EDTA (pH 8), 10% Sucrose), gewaschen. 2 g Zellen werden in 5 ml Lysozymlösung resuspendiert (0,3 M Sucrose, 25 mM Tris-HCI (pH 8), 25 mM EDTA (pH 8), 4 mg/ml Lysozym) und bei 37°C im Rundschüttier (100 rpm) inkubiert. Nach 30-45 min sind die Zellen protoplastiert. Durch Zugabe von 2,5 ml Lysemix (0,3 M NaOH, 2% Natrium-Dodecylsulfat) und sofortiges kräftiges Mischen erfolgt die Auflösung der Zellmembran und eine Denaturierung der DNA. Eine 10 minütige Hitzebehandlung (70°C) komplettiert die Lyse und Denaturierung. Bei Raumtemperatur werden 800 pl saure Phenol/Chloroform-Lösung zugegeben (Herstellung der sauren Phenol/Chloroform-Lösung: 500 ml Chloroform, 200 ml H₂0, 500 g Phenol, 0,5 g Hydroxychinolin mischen und untere Phase benutzen), um die Proteine zu denaturieren und die DNA zu renaturieren. Das Gemisch wird ca. 20 sec in einem Schüttler (®Vortex) durchmischt und dann in der Kühlzentrifuge pelletiert (15 min, 12000 rpm, 4°C).

7 ml des plasmidhaltigen Überstands werden dann in der Ultrazentrifuge weitergereinigt: 7 g CsCI, 7 ml Lysat und 0,2 ml Ethidiumbromidlösung (30 mg/ml) werden gemischt und in einer Ultrazentrifuge (^{@}Kontron TGA50) 48 h bei 34000 rpm (20°C) zentrifugiert. Die Plasmidbande wird nach UV-Bestrahlung über Fluoreszenz sichtbar gemacht und mit der Spritze entnommen. Die Lösung wird entfärbt und dialysiert und steht dann für weitere Untersuchungen zur Verfügung. Sie enthält ca. 1 pg Plasmid-DNA/20 pl Lösung.

### Beispiel 1:

### Herstellung des Plasmids pSLE40 (als Beispiel eines modifizierten Plasmids der pBR-Reihe):

pBR325-DNA kann aus plasmidtragenden Zellen nach den bekannten Verfahren (Maniatis et al.) gewonnen werden. Das Plasmid plJ6 kann aus Streptomyces lividans TC14 mit den bekannten Streptomyceten-Techniken isoliert werden (siehe Isolierung von pSG5, pSG2 oder z.B. Thompson et al., Nature 286 (1980) 525 oder US-Patentschrift 4 360 597).

Zur Klonierung wird das Plasmid pBR325 mit dem Restriktionsenzym BamHl linearisiert. 1 pg DNA wird im Spaltungspuffer (50 mM Tris HCI, pH 8,0, 10 mM MgCl₂, 50 mM NaCI) in Gegenwart von 1 unit BamHl (Hersteller: BRL, Neu Isenburg) 1 h bei 37°C inkubiert. Die Reaktion wird durch Phenolisierung (Maniatis et al.) gestoppt und die DNA durch Ethanolfällung gereinigt. Zur Gewinnung des DNA-Fragments, das die Thiostreptonresistenz trägt, wrid pIJ6-DNA mit Bcll geschnitten (Verfahren wie oben, mit der Änderung Bcll statt BamHl, Inkubation 50°C statt 37°C). Eine anschließende Behandlung von pBR325 mit alkalischer Phosphatase (aus Kälberdarm, Hersteller: Boehringer Mannheim) entfernt die 5'-Phosphatenden der DNA und verhindert die Religierung von pBR325 (Maniatis et al.). Die beiden DNA-Proben (im Spaltungspuffer) werden gemischt (0,1 µg pBR325 und 1 µg pIJ6) und auf 70°C erhitzt, um H-Brücken zu öffnen. Durch Zugabe von Mercaptoethanol (Endkonz. 10 mM) und ATP (0,1 mM) werden die Reaktionsbedingungen eingestellt. Die DNA wird in Gegenwart von 1 unit T4-DNA-Ligase (Boehringer Mannheim) bei 4°C für 12 h inkubiert. Mit dem DNA-Gemisch wird E. coli transformiert (Maniatis et al.) und die Zellen auf Chloramphenicolresistenz und Tetracyclinsensitivität selektioniert.

Einzelkolonien werden einer Schnellyse (T. Eckhardt, Plasmid 1 (1978) 584) zur Größenbestimmung unterworfen. Es ist bekannt, daß das Thiostreptonresistenzgen auf einem 1,1 kb langen Bcll-Fragment des plJ6-Plasmids lokalisiert ist (Kieser et al., Mol. Gen. Genet. 185 (1982) 223), d.h. im Agarosegel wird ein Plasmid der Größe 6,9 kb (pBR325 5,8 kb+Thiostrepton-Resistenzgen (Thio') 1,1 kb) gesucht. Aus Zellen, die ein Plasmid der geforderten Länge tragen, wird DNA isoliert (s. oben) und durch eine Restriktionsanalyse geklärt, ob das richtige DNA-Fragment inseriert ist. Restriktionsschnittstellen des Fragments sind bekannt (Kieser et al.), so daß die erfolgreiche Klonierung verfiziert werden kann.

### Beispiel 2:

### Herstellung des Pendelvektors pSW311.

Das Plasmid pSLE11 kann aus E. coli nach den bekannten Verfahren (Maniatis et al.) gewonnen werden. Das Plasmid pSG5 wird aus S. ghanaensis isoliert wie oben beschrieben.

Zur Klonierung werden beide Plasmide parallel mit EcoRl linearisiert. 1 µg DNA wird im Spaltungspuffer in Gegenwart von 1 unit EcoRl (Hersteller: Boehringer Mannheim) 1 h bei 37°C inkubiert. Die Reaktion wird durch Phenolisierung gestoppt, die DNA durch Ethanolfällung gereinigt (Maniatis et al.).

pSLE11-DNA wird zweckmäßigerweise noch mit alkalischer Phosphatase behandelt, um die Religierung zu unterbinden (Maniatis et al.).

Die DNA-Proben (im Spaltungspuffer) werden dann gemischt (0,2 µg pSLE11, 1 µg pSG5), auf 70°C erhitzt und durch Zugabe von Mercaptoethanol (Endkonz. 10 mM) und ATP (0,1 mM) auf die Ligase-Reaktionsbedingungen eingestellt. In Gegenwart von 1 unit T4-DNA-Ligase (Boehringer Mannheim) wird das Gemisch 12 h bei 4°C inkubiert.

Anschließend wird das Gemisch nach E. coli transformiert (Maniatis et al.) und auf Kolonien mit Ampicillinresistenz und Chloramphenicolsensitivität selektioniert. Kolonien mit entsprechendem Resistenzmuster werden einer Schnellyse zur Größenbestimmung unterworfen. Aus Zellen, die ein Plasmid der geforderten Länge (24,3 kb) enthalten, wird Plasmid-DNA isoliert und durch Restriktionsanalyse geklärt, ob der gewünschte Pendelvektor pSW311 vorliegt.

In analoger Weise können die in der Tabelle 3 genannten Hybridplasmide hergestellt werden.

### Beispiel 3:

### Herstellung des Plasmids pSLE41 (als Beispiel eines modifizierten Plasmids der pAC-Reihe): Konstruktion von pSLE41

pACYC184-DNA kann aus plasmidtragenden Zellen nach den bekannten Verfahren (Maniatis et al.) gewonnen werden. Wildtyp-E. co/i-Zellen besitzen allerdings eine Dam-Methylase, die die DNA so modifizieren, daß die erforderliche Restriktion mit Bc/I nicht möglich ist. Zur DNA-Isolierung wird in diesem Falle also eine Dam--Mutante von E. coli verwendet.

Das Plasmid plJ6 kann aus Streptomyces lividans TCI4 nach den bekannten Streptomyceten-Techniken isoliert werden (siehe oben).

Zur Klonierung wird das Plasmid pACYC184 mit dem Restriktionsenzym Bc/I linearisiert.

1 pg DNA wird im Spaltungspuffer in Gegenwart von 1 unit Bc/I (Hersteller: BRL, Neu-Isenburg) 1 h bei 50°C inkubiert. Die Reaktion wird durch Phenolisierung gestoppt und die DNA durch Ethanolfällung gereinigt. Eine anschließende Behandlung mit alkalischer Phosphatase (aus Kälberdarm, Hersteller: Boehringer Mannheim) entfernt die 5'-Phosphatenden der DNA und verhindert die Religierung von pACYC184. Zur Gewinnung des DNA-Fragments, das die Thiostreptonresistenz trägt, wird pIJ6-DNA mit Bc/l geschnitten (Verfahren s.o.).

Die beiden DNA-Proben (im Spaltungspuffer) werden gemischt (0,1 pg pACYC184, 1 µg pIJ6) und auf 70°C erhitzt, um H-Brücken zu öffnen. Durch Zugabe von Mercaptoethanol (Endkonz. 10 mM) und ATP (0,1 mM) werden die Reaktionsbedingungen eingestellt. Die DNA wird in Gegenwart von 1 unit T4-DNA-Ligase (Boehringer Mannheim) bei 4°C für 12 h inkubiert.

Mit dem DNA-Gemisch wird E. coli transformiert (Maniatis et al.) und die Zellen auf Tetracyclin- und Chloramphenicolresistenz selektioniert.

Einzelkolonien werden einer Schnellyse zur Größenbestimmung unterworfen. Im Agarosegel wird ein Plasmid der Größe 5,4 kb (pACYC184 4,3 kb+Thio' 1,1 kb) gesucht. Aus Zellen, die ein Plasmid der geforderten Länge tragen, wird DNA isoliert (s. oben) und durch eine Restriktionsanalyse geklärt, ob das richtige DNA-Fragment inseriert ist. Die Restriktionsschnitte des Fragments sind bekannt (Kieser et al.), so daß die erfolgreiche Klonierung verifiziert werden kann.

### Beispiel 4:

### Herstellung des Plasmids pSW344E

Das Plasmid pSLE41 kann aus E. coli nach den bekannten Verfahren (Maniatis et al.) gewonnen werden. Das Plasmid pSG5 wird aus S. ghanaensis 2932 isoliert wie oben beschrieben.

Zur Klonierung werden beide Plasmide parallel mit EcoRl linearisiert. 1 ug DNA wird im Spaltungspuffer in Gegenwart von 1 unit EcoRl (Hersteller: Boehringer Mannheim) 1 h bei 37°C inkubiert. Die Reaktion wird durch Phenolisierung gestoppt und die DNA durch Ethanolfällung gereinigt. pSLE41-DNA wird zweckmäßigerweise noch mit alkalischer Phosphatase behandelt, um die Religierung zu unterbinden. Die DNA-Proben (im Spaltungspuffer) werden dann gemischt (0,2 µg pSLE41, 1 µg pSG5), auf 70°C erhitzt und durch Zugabe von Mercaptoethanol (Endkonz. 10 mM) und ATP (0,1 mM) auf die Ligase-Reaktionsbedingungen eingestellt. In Gegenwart von 1 uniₜ T4-DNA-Ligase (Boehringer Mannheim) wird das Gemisch 12 h bei 4°C inkubiert. Anschließend wird das Gemisch nach E. co/itransformiert (Maniatis et al.) und auf Kolonien mit Tetracyclinresistenz und Chloramphenicolsensitivität selektioniert.

Kolonien mit enstprechendem Resistenzmuster werden einer Schnellyse zur Größenbestimmung unterworfen. Aus Zellen, die ein Plasmid der geforderten Länge (18,1 kb) enthalten, wird DNA isoliert und durch Restriktionsanalyse geklärt, ob die DNA das entsprechende Restriktionsmuster liefert, das bei einer Ligierung von pSLE41 und pSG5 gefordert werden muß.

In analoger Weise können die in der Tabelle 5 genannten Hybridplasmide hergestellt werden.

### Beispiel 5:

Verfährt man gemäß Beispiel 4, ligiert aber das im folgenden beschriebene, mit BamHl linearisierte "Minimalreplicon" von pSG5 mit BamHl-geschnittenem pSLE41, so erhält man den Shuttle-Vektor pGM101.

### Konstruktion eines Minimalreplikons aus pSG5

Das Plasmid pSG5 wird aus S. ghanaensis DSM 2932 wie oben beschrieben isoliert. Die Plasmide pSLE40 und pSLE41 können aus E. coli nach den bekannten Verfahren (Maniatis et al.) gewonnen werden.

In pSLE40 bzw. pSLE41 werden Fragmente von pSG5 kloniert, und zwar die Sphl-, BamHl- und Bg/II-Fragmente in die Sphl-, BamHl- und BamHI-Schnittstelle des pSLE41 und die Pstl-Fragmente in die Pstl-Schnittstelle des pSLE40. Zur Klonierung werden beide Plasmide (je ein pSLE-Plasmid und pSG5) parallel mit den obengenannten Enzymen linearisiert. Die Reaktion wird durch Phenolisierung gestoppt und die DNA durch Ethanolfällung gereinigt (Maniatis et al.).

pSLE40- und pSLE41-DNA wird zweckmäßigerweise noch mit alkalischer Phosphatase behandelt, um die Religierung zu unterbinden (Maniatis et al.).

Die DNA-Proben (im Spaltungspuffer) werden dann gemischt (0,2 µg pSLE40 bzw. pSLE41, 1 pg pSG5), auf 70°C erhitzt und durch Zugabe von Mercaptoethanol (Endkonz. 10 mM) und ATP (0,1 mM) auf die Ligase-Reaktionsbedingungen eingestellt. In Gegenwart von 1 unit T4-DNA-Ligase (Boehringer Mannheim) wird das Gemisch 12 h bei 4°C inkubiert. Anschließend wird das Gemisch nach E. coli transformiert (Maniatis et al.) und bei Verwendung von pSLE41 auf Kolonien mit Chloramphenicolresistenz und Tetracyclinsensitivität selektioniert. pSLE40-Hybride werden auf Chloramphenicolresistenz und Ampicillinsensitivität getestet. Kolonien mit entsprechendem Resistenmuster werden einer Schnellyse zur Größenbestimmung (T. Eckhardt, Plasmid 1 (1978) 584) unterworfen. Aus Zellen, die ein Plasmid der geforderten Länge enthalten, wird DNA isoliert und durch Restriktionsanalyse geklärt, ob die erwarteten Fusionsplasmide entstanden sind.

Diese Plasmide werden mit den üblichen Verfahren nach S. lividans TK 23 transformiert (K. F. Chater, D. A. Hopwood, T. Kieser und C. J. Thompson: Gene Cloning in Streptomyces, Current Topics in Microbiol. and Immunol. 96, 69-95 (1982)) und auf Thiostreptonresistenz selektioniert. Aus thiostreptonresistenten S. lividans-Kolonien isoliert man Plasmid-DNA mit Standardprozeduren (T. Kieser, Factors Affecting the Isolation of ccc DNA from Streptomyces lividans and E. coli, Plasmid 12, 19 (1984)) und prüft ihre Zusammensetzung.

Alle Plasmide, die aus thiostreptonresistenten Kolonien erhalten werden, tragen die Gene des pSG5-Plasmids, die zur Replikation benötigt werden. So läßt sich aus dem Gesamtergebnis ein zweckmäßiges "Minimalreplikon" identifizieren (hier das 4,45 kb SamHI-Fragment), das durch andere Klonierungsexperimente und durch vergleichende überlappende Klonierung weiter minimiert werden kann.

## Patentansprüche (Patentansprüche für folgende(n) Vertragsstaat(en) : BE, CH, DE, FR, GB, IT, LI, LU, NL, SE)

1. Gruppe von mindestens zwei in Streptomyceten kompatiblen Pendelvektoren mit einem E. coli-Replicon und unterschiedlichen sich von den Plasmiden pSG2, pSG5 (erhältlich aus Streptomyces ghanaensis DSM 2932) und pSVH1 ableitenden Streptomyceten-Replicons.

2. Gruppe nach Anspruch 1, in der das E. coli-Replicon sich von einem Plasmid der Reihe pBR322, pACYC177 oder pACYC184 ableitet.

3. Verwendung von mindestens zweien der Replicons aus der Gruppe der Streptomyceten-Plasmide pSG2, pSG5 (erhältlich aus Streptomyces ghanaensis DSM 2932) und pSVH1 zur Konstruktion von untereinander kompatiblen Pendelvektoren mit einem E. coli-Replikon.

## Patentansprüche (Patentansprüche für folgende(n) Vertragsstaat(en) : AT)

Verwendung von mindestens zweien der Replicons aus der Gruppe der Streptomyceten-Plasmide pSG2, pSG5 (erhältlich aus Streptomyces ghanaensis DSM 2932) und pSVH1 zur Konstruktion von untereinander kompatiblen Pendelvektoren mit einem E. coli-Replikon.

## Revendications (Revendications pour l'(les) Etat(s) contractant(s) suivant(s) : BE, CH, DE, FR, GB, IT, LI, LU, NL, SE)

1. Groupe d'au moins deux vecteurs navette compatibles dans des Streptomyces, comportant un réplicon de E. coli et des réplicons distincts de Streptomyces, dérivés des plasmides pSG2, pSG5 (pouvant être obtenus à partir de Streptomyces ghanaensis DSM 2932) et pSVH1.

2. Groupe selon la revendication 1, dans lequel le réplicon de E. coli dérive d'un plasmide appartenant à la série pBR322, pACYC177 ou pACYC184.

3. Utilisation d'au moins deux des réplicons provenant du groupe des plasmides de Streptomyces pSG2, pSG5 (pouvant être obtenus à partir de Streptomyces ghanaensis DSM 2932) et pSVH1, pour la construction de vecteurs navette compatibles entre eux, comportant un réplicon de E. coli.

## Revendications (Revendications pour l'(les) Etat(s) contractant(s) suivant(s) : AT)

Utilisation d'au moins deux des réplicons provenant du groupe des plasmides de Streptomyces pSG2, pSG5 (pouvant être obtenus à partir de Streptomyces ghanaensis DSM 2932) et pSVH1, pour la construction de vecteurs navette compatibles entre eux, comportant un réplicon de E. coli.

## Claims (Claims for the following Contracting State(s) : s: BE, CH, DE, FR, GB, IT, LI, LU, NL, SE)

1. A group of at least two shuttle vectors which are compatible in Streptomycetes and have an E. Coli replicon and various Streptomycetes replicons deriving from the plasmids pSG2, pSG5 (obtainable from Streptomyces ghanaensis DSM 2932) and pSVH1.

2. A group as claimed in claim 1, in which the E. coli replicon derives from a plasmid of the series pBR322, pACYC177 or pACYC184.

3. The use of at least two of the replicons from the group of Streptomycetes plasmids pSG2, pSG5 (obtainable from Streptomyces ghanaensis DSM 2932) and pSVH1 for the construction of mutually compatible shuttle vectors with an E. coli replicon.

## Claims (Claims for the following Contracting State(s) : AT)

The use of at least two of the replicons from the group of Streptomycetes plasmids pSG2, pSG5 (obtainable from Streptomyces ghanaensis DSM 2932) and pSVH1 for the construction of mutually compatible shuttle vectors with an E. coli replicon.
